(19) Europäisches Patentamt
European Patent Office
Office européen des brevets

(11) EP 2 133 323 B1

(12) # EUROPEAN PATENT SPECIFICATION

(45) Date of publication and mention of the grant of the patent:
**15.03.2017 Bulletin 2017/11**

(51) Int Cl.:
*C07C 69/732* (2006.01)

(21) Application number: **08425406.9**

(22) Date of filing: **09.06.2008**

(54) **The production and use of 3,5-dicaffeoyl-4-malonylquinic acid**

Herstellung und Verwendung von 3,5-Dicaffeoyl-4-Malonyl-Chinasäure

Production et utilisation de l'acide 3,5-dicaffeoyl-4-malonyl-quinique

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**16.12.2009 Bulletin 2009/51**

(73) Proprietor: **I.R.B. Istituto Di Ricerche Biotecnologiche S.r.l.
36077 Altavilla Vicentina (VI) (IT)**

(72) Inventors:
- **Pressi, Giovanna
36077 Altavilla Vicentina, Vicenza (IT)**
- **Minghetti, Anacleto
36077 Altavilla Vicentina, Vicenza (IT)**
- **Crespi Perellino, Nicoletta
36077 Altavilla Vicentina, Vicenza (IT)**
- **Boninsegna, Sara
36077 Altavilla Vicentina, Vicenza (IT)**
- **Dal Toso, Roberto
36077 Altavilla Vicentina, Vicenza (IT)**

(56) References cited:
**EP-A- 1 027 878**

- **DATABASE CA [Online] CHEMICAL ABSTRACTS SERVICE, COLUMBUS, OHIO, US; ZHANG, YUFENG ET AL: "Characterization of phenolic compounds in Erigeron breviscapus by liquid chromatography coupled to electrospray ionization mass spectrometry" XP002507802 retrieved from STN Database accession no. 2007:1087203 & RAPID COMMUNICATIONS IN MASS SPECTROMETRY , 21(18), 2971-2984 CODEN: RCMSEF; ISSN: 0951-4198, 2007,**
- **MAI THANH THI NGUYEN ET AL: "Xanthine Oxidase Inhibitory Activity of Vietnamese Medicinal Plants" BIOL. PHARM. BULL., vol. 27, no. 9, 2004, pages 1414-1421, XP009109766**
- **HONG LIU ET AL: "Effects of Erigeron breviscapus ethanol extract on neuronal oxidative injury induced by superoxide radical" FITOTERAPIA, vol. 76, 2005, pages 666-670, XP002507800**
- **SATAKE TOSHIKO ET AL.: "The anti-thrombotic active constituents from Centella asiatica" BIOLOGICAL & PHARMACEUTICAL BULLETIN, vol. 30, no. 5, 2007, pages 935-940, XP009109763**
- **J.M.SOLET ET AL: "Centella asiatica (L.) Urban. (Pennywort): Cell Culture, Production of Terpenoids, and Biotransformation Capacity", BIOTECHNOLOGY IN AGRICULTURE AND FORESTRY, VOL 41 MEDICINAL AND AROMATIC PLANTS X, 1998, XP009188607,**
- **Anna Ling Pick Kiong: "Effects of Precursor Supplementation on the Production of Triterpenes by Centella asiatica Callus Cultures", Pakistan Journal of Biological Sciences 8(8), 1 January 2005 (2005-01-01), pages 1160-1169, XP55250950, Retrieved from the Internet: URL:http://docsdrive.com/pdfs/ansinet/pjbs /2005/1160-1169.pdf [retrieved on 2016-02-17]**
- **Rozita Omar ET AL: "Development of Growth Medium for Centella Asiatica Cell Culture Via Response Surface Methodology", American Journal of Applied Sciences, vol. 1, no. 3, 1 March 2004 (2004-03-01), pages 215-219, XP55044968, ISSN: 1546-9239, DOI: 10.3844/ajassp.2004.215.219**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **Rozita Omar: "Optimization and Elucidation of Interactions between Ammonium, Nitrate and Phosphate in Centella asiatica cell Culture Using Response Surface Methodology", Biotechnology and Bioprocess Engineering 10, 1 January 2005 (2005-01-01), pages 192-197, XP55251082, Retrieved from the Internet: URL:http://download.springer.com/static/pdf/819/art%3A10.1007%2FBF02932012.pdf?originUrl=http://link.springer.com/article/10.1007/BF02932012&token2=exp=1455726233~acl=/ static/pdf/819/art%253A10.1007%252FBF02932012.pdf?originUrl=http%3A%2F%2Flink.springer.com%2Farticle%2F10.1007%2FBF02932012*~h mac=b7def5 [retrieved on 2016-02-17]**
- **Rozita Omar: "Kinetics and Modelling of Cell Growth and Substrate Uptake in Centella asiatica Cell Culture", Biotechnology and Bioprocess Engineering 11, 1 January 2006 (2006-01-01), pages 223-229, XP55251084, [retrieved on 2016-02-17]**


Remarks:

The file contains technical information submitted after the application was filed and not included in this specification

## Description

Field of the invention

The present invention refers to the attainment of a natural caffeine derivative 3,5-dicaffeoyl-4-malonylquinic acid, and discloses its use in human and veterinary therapy and for nutritional, cosmetic and pharmaceutical purposes.

State of the art

*Centella asiatica* is a small perennial plant, belonging to the Apiaceae family and found in India, China, Indonesia, Australia, Madagascar, Central and Southern Africa; it grows in very humid regions at altitudes above 700 metres above sea level. This plant species is used in traditional Indian medicine to treat various pathologies, in particular wound healing (scar formation). *Centella asiatica* is listed in the official Chinese Pharmacopeia, and is used as an antipyretic, diuretic, to treat ulcers, eczemas and trauma (Kartning T., Herbs, Spices and medicinal plants, vol.3, L.E. Cracker, J.E. Simmons Eds., Oryx Press Arizona, USA, 1998).

Currently, commercially prepared extracts of said plant are widely used in the treatment of chronic venous insufficiency and skin ailments. In particular, commercially prepared extracts are titred in **triterpenes** (asiaticoside, asiatic acid, madecassic acid and other triterpenes). Numerous publications in the literature (Belcaro G. et al., 1990, Angiology, 41:12; Belcaro G. et al., 1990, Curr. Ther. Res., 47:421) describe that triterpenes of *Centella asiatica* are effective in counteracting chronic venous and microcirculation insufficiency, through improving microcirculation and the metabolic activity of vascular and perivascular connective tissue.

Caffeine derivatives are polyphenol compounds representing a group of secondary metabolites found widely in higher plants. In recent years, these compounds have received special attention since they protect the body against oxidative stress, believed to be one of the causes of the onset of cancer, cardiovascular and neurodegenerative disorders (Toyokuni S. et al., FEBS Letters, 358(1995):1-3; Ochiai T. et al., Neuroscience Letters, 362(2004):61-64; Mertens et al., Circulation, 107(2003: 1640-1646).

With regard to their high antioxidant activity, said compounds are widely used in numerous pathologies associated with damage due to free radicals.

It is known from the scientific literature that molecules with antioxidant activity play a fundamental role in the prevention of vascular disorders and other chronic inflammatory pathologies (Salvemini D., Cuzzocrea S., 2002, Free Radical Biology & Medicine, 33-9:1173-1185). Thanks to their antioxidant and radical scavenging activities, these molecules are capable of counteracting the oxidation of low density lipoprotein (LDL) and inhibiting platelet aggregation (Yann C. et al., 2005, Pharmacological reports, 57:97-107). Furthermore, said compounds are capable of modulating **nitric oxide (NO)** generation by vascular endothelium and interfering with the mechanisms leading to inflammation and endothelial apoptosis, contributing towards preventing endothelial malfunctions, which are known to play a crucial role in the pathogenesis of venous insufficiency.

It is well known in the literature that the formation of oxygen radicals/reactive oxygen species (ROS) is associated with the inflammatory reaction and frequently their presence contributes towards inducing tissue damage (Pawliczak R., 2003, Pol. Merkuriusz Lek., 14:493-6; Leiro J. et al., 2004, J. Leukoc. Biol.,75).

In the scientific publication "Characterization of phenolic compounds in Erigeron breviscapus by liquid chromatography coupled to electrospray ionization mass spectrometry", Rapid Commun Mass Spectrom. 2007;21(18):2971-84, Zhang Yufeng *et al.",* the authors disclose that the 3,5-dicaffeoyl-4-malonylquinic acid has been identified in a methanol extract of the *Erigeron breviscapus* plant.

The antioxidant activity of an ethanol extract of *Erigeron breviscapus* is disclosed in the scientific article: "Effects of Erigeron breviscapus ethanol extract on neuronal oxidative injury induced by superoxide radical Fitoterapia", 2005 Dec; 76(7-8):666-70. Epub 2005 Oct 24, Hong liu *et al.*

The following prior art references disclose "classical" plant extract of *Centella asiatica* and biological properties:

Antioxidant properties of methanol extracts of the whole plant of *Centella asiatica* are disclosed in the scientific publication "Xanthine oxidase inhibitory activity of Vietnamese medicinal plants", Biol Pharm Bull. 2004 Sep; 27(9):1414-21, Mai Thanh Thi Nguyen *et al.*

Anti-thrombotic activity of methanol extracts from the aerial parts of *Centella asiatica* are disclosed in "The antithrombotic active constituents from Centella asiatica", Biol Pharm Bull. 2007 May; 30(5):935-40, Satake Toshiko *et al.*

EP-A-1 027 878 is mentioning a total extract of *Centella asiatica* as an example of lipophilic active ingredient that can be incorporated in oily globules of a cosmetic oil in water emulsion.

The following prior art references disclose plant cell culture extracts of *Centella asiatica.*

The scientific publication "Centella asiatica (L.) Urban, (Pennywort): Cell Culture, Production of Terpenoids, and Biotransformation Capacity", Biotechnology in Agriculture and Forestry, Volume 41, pp 81-96, J. M. Solet *et al.,* discloses *in vitro* cell culture of *Centella asiatica* and the biotransformation of papaverine and thiocolchicine by *Centella*

*asiatica* cell suspension.

The scientific publication "Effects of Precursor Supplementation on the Production of Triterpenes by Centella asiatica Callus Cultures", Pakistan Jownal of Biological Sciences 8 (8): 1160-1169, 2005, Anna Ling Pick Kiang *et al.,* discloses the production of four targeted triterpenes, asiatic acid, madecassic acid, asiaticoside and madecassoside in leaf dried derived callus and cell suspension cultures of *Centella asiatica.*

The scientific publication "Development of Growth Medium for Centella Asiatica Cell Culture via Response Surface Methodology", American Journal of Applied Sciences 1(3): 215-219, 2004, Rozita Omar *et al.,* discloses a study of the effect of sucrose, indole-3-acetic acid and 6-benzylaminopurine concentrations on cell growth of *Centella asiatica* cell suspension culture.

The scientific publication "Optimization and elucidation of interactions between ammonium, nitrate and phosphate in Centella asiatica cell culture using response surface methodology", Biotechnology and Bioprocess Engineering, June 2005, Volume 10, Issue 3, pp 192-197; Rozita Omar *et al.,* discloses the effects of macronutrients on cell growth and the triterpenoids production in *Centella asiatica* cell suspension cultures.

The scientific publication "Kinetics and modelling of cell growth and substrate uptake in Centella asiatica cell culture", Biotechnology and Bioprocess Engineering, June 2006, Volume 11, Issue 3, pp 223-229, Rozita Omar *et al.,* discloses kinetics and modelling studies to evaluate cell growth for a better understanding and control of the process. Triterpenoids production in *Centella asiatica* cell suspension cultures are mentioned.

None of these scientific publications discloses the presence of 3,5-dicaffeoyl-4-malonylquinic acid in *Centella asiatica* cell culture extracts.

## Sunimary of the invention

The invention is based on the isolation of the caffeine derivative, 3,5-dicaffeoyl-4-malonylquinic acid, extracted as a metabolite from *Centella asiatica* cell cultures, as will be described hereinafter.

A first object of the invention is *Centella asiatica* cell culture extracts comprising 3,5-dicaffeoyl-4-malonylquinic acid as recited in claim 1.

The compound 3,5-dicaffeoyl-4-malonylquinic acid has been observed to be endowed with marked antioxidant, anti-inflammatory, antimicrobial, photoprotective, depigmenting, antiviral and metalloproteinase inhibitory activities.

Another object of the invention is a process for the production of 3,5-dicaffeoyl-4-malonylquinic acid, from *Centella asiatica* cell cultures, as recited in claim 2, also on the industrial scale.

3,5-dicaffeoyl-4-malonylquinic acid, particularly for use in drugs, nutritional products, cosmetics and for zootechnic use, is disclosed hereafter.

Further advantages of the present invention will become clearer from the following detailed description of the invention.

## Brief description of the figures

**Figure 1** shows the HPLC chromatogram of *Centella asiatica* extract;
**Figure 2** shows the anti-HSV2 antiviral activity of 3,5-dicaffeoyl-4-malonylquinic acid;
**Figure 3** shows the metalloproteinase inhibitory activity of 3,5 dicaffeoyl-4-malonylquinic acid.

## Detailed description of the invention

3,5-dicaffeoyl-4-malonylquinic acid (Molecular weight: 602 Daltons, empirical formula: $C_{28}H_{26}O_{15}$) has been isolated from selected cell culture extracts of *Centella asiatica* and its structure has been confirmed by mass spectrometry (MS) and nuclear magnetic resonance (NMR) spectroscopy. The structural formula (I) of the compound is as follows:

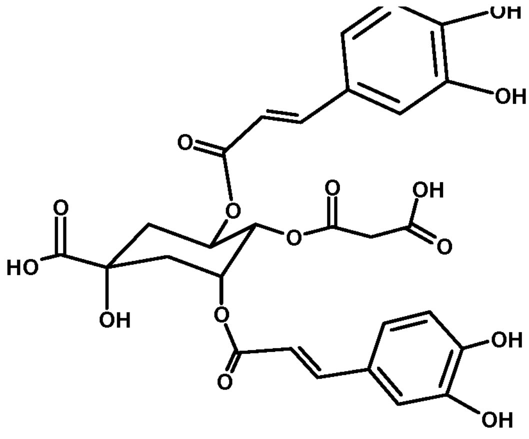

(I)

An object of the invention is therefore the process for the preparation of this compound, the solvates and salts thereof with pharmaceutically acceptable bases.

The process for the preparation of said compound envisages extraction from *Centella asiatica* cell culture biomass. The method of the invention, allowing the attainment of the compound in such quantities as to even justify industrial production, comprises the attainment *Centella asiatica* plant tissue calluses, stabilisation of the cell line obtained from callus tissue, the selection of a cell line with high 3,5-dicaffeoyl-4-malonylquinic acid content (obtained by means of standard procedures reported in the literature: Schmauder H.P., Doebel P., Acta Biotechnolo., 1990; 10: 501-516; or as will be described hereinafter), transfer of the stabilised and selected cell line to liquid culture medium and the production of high quantities of biomass in suitable fermenters.

An extraction of said selected plant cell line has been conducted and described below, using the procedure discussed later.

### Attainment of a *Centella asiatica* cell culture

This process envisages the collection of plant tissue, the cleaning thereof, for example under running water, cutting into fragments of 2-5 cm and sterilisation on plates, for example by sequential treatment with 70% ethanol for approx. 15 minutes, 2% sodium hypochlorite for approx. 5 minutes and finally 0.05% $HgCl_2$ for approx. 1 minute. Between treatments, the plant fragments are washed, typically three or more times with sterile distilled water.

Each fragment of said tissue, chopped-up even further (explants), is placed on a Petri dish containing nutrient medium, solidified by the addition of Agar and supplemented with growth hormones without the addition of any antibiotics. The number of explants performed influences the outcome of the subsequent stages. Generally from 2000 to 5000 uncontaminated explants are sufficient to proceed to the subsequent selection stage.

Following a suitable period of time, undifferentiated callus tissue forms, which is then multiplied following transfer onto a greater surface area with fresh medium.

Furthermore, the plant cell culture derived from the undifferentiated callus tissue is preferably stabilised by means of a certain number of transfers (sub-cultures) onto fresh culture medium. Particularly, it has been observed that in order to obtain a stable cell culture, it is important to perform at least ten sub-cultures. Such medium is solid in nature, and may be advantageously constituted by 0.8-1% agar in a standard culture medium, to which has been added plant peptone, allowing a balanced supply of aminoacids and guaranteeing the maintenance of good cell wall integrity. Preferably, plant peptone will be added in quantities ranging between 500 and 4000 mg/l of culture medium.

A "stable cell culture" is defined as a culture having the following characteristics:

- high and constant proliferation rate over time;
- preservation of the same phenotypic characteristics throughout various subcultures (cell colour, aggregate friability, size etc.);
- constant secondary metabolite levels per unit of mass, over the course of the various subculture steps (secondary metabolite content is assessed by chemical analysis of the extracts);
- constant primary metabolite content (protein, lipids and polysaccharides) per unit mass.

After the stabilisation stage, the cell culture is subjected to "clonal selection". Such selection consists of growing

the stabilised cells for an appropriate amount of time (typically, 10-15 days of culture). Subsequently, individual cell aggregates are taken from the solid culture medium and each of said cell aggregates is inoculated into liquid culture medium described above.

Following fermentation for such time necessary to obtain adequate multiplication of the cell aggregate (hereinafter referred to as "clone"), a time generally comprised of between 10 and 15 days, the content of the metabolite of interest is determined for each clone.

Such operations may be repeated until a plant cell culture clone is selected, in which production of the metabolite of interest is the highest.

It should be remembered that alternating periods of culture on solid and liquid media is essential for the clonal selection method of the present invention. Hence, it is essential that the clonal selection process described above does not conclude with the identification of the most active clone, but is constantly repeated so as to keep the selected clone phenotypically homogeneous.

The selected plant cell culture is then multiplied in order to obtain a sufficient quantity of biomass to carry out the production fermentation stage. Said quantity will depend on the specific production requirements.

The biomass thus obtained may be passed directly into the final fermenter, or can be subjected to one or more further growth stages in liquid medium, working with intermediate volumes.

Preferably, the process just illustrated includes the stages of:

a) cultivating a predetermined plant cell culture, stabilised for a sufficient period of time to allow the multiplication of said cell culture to give substantially distinct cell clusters, on solid medium;
b) removing said substantially distinct cell clusters from said solid medium and placing each of them in a separate liquid culture medium;
c) cultivating each of the said substantially distinct cell clusters in said liquid culture medium for a sufficiently long period of time to allow the multiplication of said cell clusters, and the analytical determination of the primary and/or secondary metabolites produced thereby;
d) performing a qualitative and quantitative determination of the primary and/or secondary metabolites produced by each of said cell clusters in said liquid culture medium;
e) selecting the cell cluster capable of producing the greatest quantity of said metabolite of interest;
f) repeating the process cycle according to stages a), b), c), d) and e) on said cell cluster, selected according to stage e), a sufficient number of times until the quantity of said metabolite of interest produced by a selected cell cluster, and by the cell clusters deriving from further selection cycles, is essentially constant.

In addition, the subsequent fermentation may preferably comprise the following stages:

A) the inoculation of said plant clone into liquid medium and the multiplication thereof for a sufficient period of time to obtain an increase in cellular mass of at least 300% of the weight thereof;
B) optionally, transfer of the suspension obtained from stage A) into fresh liquid culture medium and the multiplication thereof for a sufficient period of time to obtain an increase in cellular mass of at least 300% of the weight thereof;
C) optionally, the repetition of stage B) at least one additional time;
D) the transfer of the suspension obtained in stages A), B) or C) into fresh liquid culture medium in a fermenter to give a biomass, and conducting the fermentation under such conditions and for a sufficient period of time so as to obtain within said biomass, the production of said at least one metabolite of interest;
E) the separation of said at least one metabolite of interest from said biomass.

In accordance with one preferred embodiment, the fermentation will normally be performed at a temperature of between 15°C and 35°C, typically around 25°C and for a period of time normally between 7 and 40 days, preferably between 14 and 21 days. It is essential that the biomass be adequately aerated and that at the same time be kept stirred by means of stirring external to the fermenter. Indeed, it has been observed that plant biomass is comprised of cells having cell-walls that are poorly resistant to rupture. A stirrer submerged into the biomass acts mechanically on the cells and can easily cause the lysis thereof. However, it is necessary that the stirring, although delicate, be efficient, above all in the final fermentation stages when the biomass greatly increases in density. For the purposes of the present invention, particularly appropriate methods of stirring are orbital means of stirring. Such means of stirring preferably operate at 40-200 rpm, more preferably at about 120 rpm.

It is appropriate that the volume of the container (fermenter) in which the fermentation occurs be considerably greater than the volume of the biomass. Typically, the volume of the reactor will be from 50% to 200% greater than the biomass volume.

As already mentioned, efficient fermentation requires adequate oxygenation. Oxygenation is normally performed by using sterile air with a flow rate of 0.5-4 l/minute, more preferably 2-2.5 l/minute, for a volume of 10 litres of

biomass. Alternatively, gas mixtures containing from 10% to 100% v/v of oxygen may be used.

As mentioned previously in relation to stirring, even oxygenation by means of over violent bubbling can cause rupturing of the cell walls. Hence it is necessary to ensure that oxygenation is performed delicately, for example by bubbling through appropriate diffusers. It will be preferable to use means of air or oxygen diffusion with nozzle delivery flow speeds comprised of between 10 m/min and 600 m/min, more preferably between 50 m/min and 350 m/min.

In addition, the shape of the fermentation chamber has significant importance. Indeed, it is recommended that it has a smooth and uniform surface, i.e. that there are no edges, corners or other parts which can cause the cell walls of the biomass rupture. According to one particular embodiment of the present invention, additives increasing water oxygen solubility will be added to the biomass. Such additives will preferably be selected from those substances defined as "artificial blood", for example the perfluorinated hydrocarbons (PFC).

In particular, for the scope of the present invention, stable cell cultures derived from *Centella asiatica* have been selected for their capacity to produce 3,5-dicaffeoyl-4-malonylquinic acid in suitable qualitative and quantitative proportions.

The conditions for extraction of the compound 3,5-dicaffeoyl-4- malonylquinic acid from cell cultures will be described by way of non-limiting example.

**Extraction method**

The inventive method for the extraction of 3,5-dicaffeoyl-4-malonylquinic acid from a *Centella asiatica* cell culture includes the stages of: i) forming a homogenised suspension of said cell culture at pH 6 to 12, preferably at pH 8, ii) eliminating the corpuscular part, iii) extracting the resulting solution, at a pH of between approx. 3 and 4, with a solvent capable of dissolving 3,5-dicaffeoyl-4-malonylquinic acid, or iv) extracting the resulting solution, at a pH of between approx. 3 and 4, with hydrophobic interaction resin.

In particular, the cell culture extraction process includes the following steps in sequence:

a) filtration of the biomass obtained from fermentation of the *Centella asiatica* cell culture, so as to obtain only the cells and discard the medium;

b) the addition of an equal volume of ethanol (or any other alcohol miscible with water with from 2 to 5 carbon atoms) to the cells along with 0.1-0.8 g/l ascorbic acid, preferably 0.5 g/l, and 1.0-3.0 g/l, preferably 2.0 g/l, citric acid or any other organic or mineral acid that adjusts the pH of the suspension to values around 3 or 4;

c) homogenisation of the entire mixture, for example using an UltraTurrax or any other device capable of disrupting the cells and their internal structures;

d) removal of the cell residue by centrifugation or filtration through nylon or cotton mesh with porosity comprised of between 10 and 300 $\mu$m, preferably between 50 and 150 $\mu$m;

e) removal of the alcohol and a portion of the water from the hydroalcoholic phase of the filtrate by distillation under reduced pressure;

f) extraction of the aqueous suspension with ethyl acetate or any other solvent that is partially miscible or imiscible with water, in which 3,5-dicaffeoyl-4-malonylquinic acid is soluble;

g) separation of the organic phase and the anhydration of the same, for example, using sodium sulphate or some other dehydrating agent;

h) reduced pressure distillation of the solvent from the anhydrous organic solution until the attainment of a solid with a 3,5-dicaffeoyl-4-malonylquinic acid content in excess of 50%.

Alternatively, a second 3,5-dicaffeoyl-4-malonylquinic acid extraction system may be adopted including the following steps:

a) homogenisation of the entire culture, for example using a UltraTurrax or any other device capable of disrupting the cells and their internal structures, following the addition of 2 g/l sodium bicarbonate or another basic compound, such as for example potassium bicarbonate or sodium carbonate, capable of adjusting the pH to around 8;

b) removal of the corpuscular part from the suspension by centrifugation or filtration through nylon or cotton mesh with porosity comprised of between 10 and 300 $\mu$m, preferably between 50 and 150 $\mu$m;

c) reducing the pH of the filtrate to values around 4, for example, through the addition of citric acid or any other organic or inorganic acid;

d) extraction of the acidified solution with ethyl acetate or any other solvent that is partially miscible or imiscible with water and capable of dissolving 3,5-dicaffeoyl-4-malonylquinic acid;

e) anhydration of the organic phase, for example with sodium sulphate or any other dehydrating agent, and reduced pressure distillation of the same until the attainment of a solid.

Alternatively, the aforementioned operations up to points d) and e) may be substituted by extraction of the acidic aqueous phase obtained at point c) using lipophilic interaction resin such as those with styrene or divinylbenzene structures, preferably XAD4. Said resin may be eluted using an ethanol or methanol solution at concentrations varying between 50% and 95%, preferably 90%.

The eluate may be distilled under reduced pressure until all the ethanol is removed, and the aqueous residue lyophilised or extracted with ethyl acetate or another suitable solvent.

Using the above-cited methods, a *Centella asiatica* cell culture extract is obtained where 3,5-dicaffeoyl-4-malonylquinic acid is present in quantities exceeding 10% by weight. In one particularly preferred embodiment of the invention, the cell culture may yield an extract where 3,5-dicaffeoyl-4-malonylquinic acid is present in quantities in excess of 30% by weight, preferably in excess of 50% by weight.

By way of non-limiting example, an example of the preparation of 3,5-dicaffeoyl-4-malonylquinic acid from *Centella asiatica* cell cultures is given below.

## EXAMPLE 1

## LABORATORY SCALE PREPARATION OF 3,5-DICAFFEOYL-4-MALONYLQUINIC ACID FROM CELL CULTURES OF *CENTELLA ASIATICA*

The laboratory scale extraction of 3,5-dicaffeoyl-4-malonylquinic acid from cell cultures of *Centella asiatica* is reported by way of demonstrative but non-limiting example. Calluses grown on solid medium (GAMBORG B5 with 1% agar supplemented with 20 g/l sucrose, 1 g/l plant peptone, 1 mg/l kinetin, 1 mg/l naphthalenacetic acid and 0.2 mg/l indolacetic acid at pH 6.5) and subjected to subculture for at least three months, are inoculated into 10 x 300 ml flasks, each containing 50 ml of liquid medium (GAMBORG B5 supplemented with 20 g/l sucrose, 1 g/l plant peptone, 1 mg/l kinetin, 1 mg/l naphthalenacetic acid, 0.2 mg/l indolacetic acid at pH 6.5). After 7 days of fermentation, the cultures are used as the inoculum for the same number of 1000 ml flasks, each containing 250 ml of liquid medium. After 7 days of fermentation, the 1000 ml flasks, each containing 250 ml of culture are used as the inoculum for the same number of 3 l flasks, each containing 1 l of liquid medium.

Fermentation is monitored by means of samples taken sequentially over time and is terminated upon reaching an age of 14 days. The biomass is combined and filtered through nylon mesh with a porosity of 100 μm.

The filtrate is discarded and the cells suspended in an equal volume of ethanol supplemented with 0.5 g/l ascorbic acid and 2 g/l citric acid.

The suspension is homogenised using an UltraTurrax and then filtered. The cell residue is extracted a further twice with 96% ethanol. The combined filtrates contain 9.5 g of 3,5-dicaffeoyl-4-malonylquinic acid. All the ethanol is removed from the filtrates and the aqueous residue extracted twice with ethyl acetate.

The ethyl acetate extracts are anhydrated over sodium sulphate, filtered and dried by reduced pressure distillation. 17.5 g of a pale yellow coloured crude extract, containing 9.3 g of 3,5-dicaffeoyl-4-malonylquinic acid, is obtained. In order to obtain purer material, the extract is loaded onto an RPC18 column in 15% acetonitrile in water, and the column eluted isocratically. A chromatographic peak is obtained which, following removal of the organic solvent by distillation, and the aqueous phase by lyophilisation, leaves a residue of 7.1 g of pure 3,5-dicaffeoyl-4-malonylquinic acid, suitable for structural analysis.

## EXAMPLE 2

## INDUSTRIAL SCALE PREPARATION OF 3,5-DICAFFEOYL-4-MALONYLQUINIC ACID FROM CELL CULTURES OF *CENTELLA ASIATICA*

The industrial scale extraction of 3,5-dicaffeoyl-4-malonylquinic acid from cell cultures of *Centella asiatica* is reported by way of demonstrative but non-limiting example.

Solid sodium bicarbonate is added under agitation to 200 litres of 14 day *Centella asiatica* cell suspension (obtained using the methods described in example 1) coming from 2 fermenters, until the pH of the suspension is around 8.

The cell suspension is homogenised using an UltraTurrax and filtered through 100 μm nylon mesh or centrifuged.

The residue is extracted a further twice with equal volumes of water. The aqueous extracts are combined and the pH adjusted to around 4 by the addition of solid citric acid. The acidified extracts are then passed through a column of XAD4 resin in order to fix the 3,5-dicaffeoyl-4-malonylquinic acid. The column is then eluted with ethanol/water (80/20) until the complete release of the 3,5-dicaffeoyl-4-malonylquinicoi acid, and the eluate distilled until the ethanol is completely removed. The residual aqueous suspension is then lyophilised.

In order to obtain material with a higher grade of purity, rather than lyophilisation, the aqueous suspension is adjusted to a pH of less than 4 with solid citric acid and extracted twice with ethyl acetate.

The combined organic extracts are anhydrated over sodium sulphate, filtered and dried by reduced pressure distillation.

245 g of a yellow coloured solid, containing 154 g of 3,5-dicaffeoyl-4-malonylquinic acid, is obtained.

HPLC analysis

High pressure liquid chromatography (HPLC) analysis is performed using a Phenomenex 4.6 x 150 mm 3 μm C18 column (2), with the following eluent mixtures:

Solvent A: 0.1% $H_3PO_4$ in $H_2O$, supplemented with 0.5% solvent B
Solvent B: 0.1% $H_3PO_4$ in $CH_3CN$, supplemented with 0.5% solvent A.
Flow rate: 1 ml/min

The gradient used has been the following:

| T (min) | solv. B (%) |
|---|---|
| 0 | 20 |
| 5 | 30 |
| 7 | 40 |
| 10 | 40 |
| 15 | 20 |

Quantification of the compound has been performed at 330 nm.

Table 1 - Retention time of the compound

| Product | RT (min) |
|---|---|
| 3,5-dicaffeoyl-4-malonylquinic acid | 7.9 |

Figure 1 shows the chromatogram of *Centella asiatica* extract, containing 3,5-dicaffeoyl-4-malonylquinic acid, at 330 nm.

**EXAMPLE 3**

**THE ANTIOXIDANT ACTIVITY OF 3,5-DICAFFEOYL-4-MALONYLQUINIC ACID**

The antioxidant activity of the compound 3,5-dicaffeoyl-4-malonylquinic acid compared to known molecules such as ascorbic acid and rutin, has been determined by means of the DPPH method, according to the instructions reported in J. Buenger et al.; International Journal of Cosmetic Science, 2006, 28: 135-146. The results obtained are reported in Table 2:

TABLE 2

| Compounds | IC50 (μM) |
|---|---|
| 3,5-dicaffeoyl-4-malonylquinic acid | 3.76 |
| Ascorbic acid | 1 |
| Rutin | 13.51 |

The data obtained indicates that the compound obtained from cell cultures of *Centella asiatica* has higher antioxidant activity compared to more well known antioxidants such as ascorbic acid and rutin.

## EXAMPLE 4

### THE ANTI-INFLAMMATORY ACTIVITY OF 3,5-DICAFFEOYL-4-MALONYLQUINIC ACID ON A DNBS INDUCED COLITIS MODEL

Evaluation of the anti- inflammatory activity of 3, 5-dicaffeoyl-4-malonylquinic acid has been determined on an *in vivo* induced colitis model. Colitis has been induced in rats using a colon inflammation model, stimulated by DNBS (2,4,6 dinitrobenzenesulphonic acid). After 4 days of induction of inflammation, the following parameters have been evaluated: determination of colon damage by means of morphological and histological analysis; determination of myeloperoxidase activity and determination of body weight. The animals used have been subdivided into four experimental groups (10 Sprague-Dawley rats weighing 200-250 g in each group) :

- one control group, to which only carrier has been administered (CTR);
- one group, to which DNBS plus carrier has been administered (DNBS);
- one group, to which DNBS and 3,5-dicaffeoyl-4-malonylquinic acid has been administered: the compound has been administered orally at a concentration of 2 mg/kg for 4 consecutive days following the administration of DNBS;
- one group, to which DNBS and 3,5-dicaffeoyl-4-malonylquinic acid has been administered: the compound has been administered orally at a concentration of 0.2 mg/kg for 4 consecutive days following the administration of DNBS.

Four days after the administration of DNBS, the following investigations have been conducted:

- evaluation of colon damage by means of morphological and histological analysis (a score is assigned on the basis of the damage observed);

- evaluation of myeloperoxidase activity and

- determination of body weight.

The results obtained are summarised in table 3:

Table 3: histological score, myeloperoxidase activity and body weight

| ***Groups*** | ***Histolog ical score*** | ***Myeloperoxidase activity (U/g fresh weight)*** | ***Body weight (g)*** |
|---|---|---|---|
| Control | 0 | 66.00 | + 8.9 |
| DNBS | 3.70 | 1021.50 | - 16.6 |
| DNBS + 0.2 mg/kg 3,5-dicaffeoyl-4-malonylquinic acid | 1. 60 | 513.00 | - 7.0 |
| DNBS + 2 mg/kg 3,5-dicaffeoyl-4-malonylquinic acid | 1.55 | 486.90 | - 5.5 |

Treatment with 3,5-dicaffeoyl-4-malonylquinic acid, administered orally, significantly reduces, in a dose-dependent manner:

- the extent and severity of histological colon damage;
- the degree of infiltration of polymorphonuclear leukocytes and the reduction in body weight.

## EXAMPLE 5

### THE ANTIMICROBIAL ACTIVITY OF 3,5-DICAFFEOYL-4-MALONYLQUINIC ACID

With the aim of identifying any potential antimicrobial activity in the compound 3,5-dicaffeoyl-4-malonylquinic acid, microbiological assays, generally known as multiple dilution assays, have been prepared. The results obtained from said assays have shown the antimicrobial activity of the compound against the following strains:

- *Staphylococcus aureus ATCC6538*

- *Malassezia furfur DSMZ4167*

The compound has been tested at concentrations of 5 mg/ml, 2 mg/ml and 1 mg/ml.

The microbial strains used for testing have been inoculated into culture-broth (in Tryptone soy broth medium, for *Malassezia furfur* the culture medium has been supplemented with 2 ml/100 ml of olive oil) containing *the* test compound at the concentrations described above. In parallel with these tests, culture broths containing the microbial strain alone have been prepared (controls). The culture-broths have been incubated at 30°C for a period of 7 days. Samples of the various culture-broths have been taken at 0 and 7 days, with the aim of counting the number of CFU/ml in relation to the various microbial strains, by plating the cultures on solid medium. A certain number of serial dilutions have been prepared for each sample in order to determine the microbial load of each culture-broth. Each dilution has been assayed in triplicate on solid medium. Then number of CFUs reported in the table is the mean of the counts performed on the triplicate assays. The results obtained are reported in the following table.

Table 4: the effect of 3,5-dicaffeoyl-4-malonylquinic acid on the growth of *Staphylococcus aureus ATCC 6538*

| **Samples** | **TO (CFU/ml)** | **T7d (CFU/ml)** |
|---|---|---|
| Control | $1.9 \times 10^5$ | $2.2 \times 10^5$ |
| 3,5-dicaffeoyl-4-malonylquinic acid (5mg/ml) | $1.9 \times 10^5$ | <100 |
| 3,5-dicaffeoyl-4-malonylquinic acid (2mg/ml) | $1.9 \times 10^5$ | $3.8 \times 10^4$ |
| 3,5-dicaffeoyl-4-malonylquinic acid (1mg/ml) | $1.9 \times 10^5$ | $2.3 \times 10^5$ |

Table 5: the effect of 3,5-dicaffeoyl-4-malonylquinic acid on the growth of *Malassezia furfur DSMZ4167*

| **Samples** | **TO (CFU/ml)** | **T7d (CFU/ml)** |
|---|---|---|
| Control | $9.2 \times 10^3$ | $2.8 \times 10^4$ |
| 3,5-dicaffeoyl-4-malonylquinic acid (5 mg/ml) | $9.2 \times 10^3$ | <100 |
| 3,5-dicaffeoyl-4-malonylquinic acid (2 mg/ml) | $9.2 \times 10^3$ | <100 |
| 3,5-dicaffeoyl-4-malonylquinic acid (1 mg/ml) | $9.2 \times 10^3$ | $8.8 \times 10^3$ |

From the results obtained, it is evident that 3,5-dicaffeoyl-4-malonylquinic acid at a concentration of 5 mg/ml exerts antimicrobial activity towards *Staphylococcus aureus ATCC6538,* and at a concentration of 2 mg/ml, exerts antimicrobial activity towards *Malassezia furfur DSMZ4167.*

**EXAMPLE 6**

**THE SUN-SCREEN ACTIVITY OF 3,5-DICAFFEOYL-4-MALONYLQUINIC ACID**

This example reports the test to determine the UV absorption of the molecule 3,5-dicaffeoyl-4-malonylquinic acid compared to a commercially available molecule widely used as a sun screen (PARSOL MCX).

The compounds have been examined in an aqueous solution of:

0.2 M phosphate buffer pH 5.0
0.1 M HCl solution at pH 1.0.

The pH 5.0 buffer has been selected as the condition used most frequently in the preparation of sun screens. The pH 1.0 solution has been examined in order to also observe the stability of the compounds at extreme pH values.

The analyses have been conducted using a Shimadzu model UV 1601 spectrophotometer, analysing the spectrum from 250 nm to 400 nm in order to ascertain the absorption of UVA and UVB.

The results report the absorption values at maximum wavelength ($\lambda$ Max) and at 330 nm, this being the wavelength most frequently used for quantitative measurements for the products in question.

The values are expressed in:

- specific extinction, corresponding to the extinction at the given $\lambda$ for a solution at a concentration of 1.0 $\mu$g/mL.

- Epsilon = the molar absorption, calculated using the formula

$$Epsilon= A/ C*L$$

where A is the absorption value in mAU, C is the concentration of the solution in moles and L is the path length of the solutions in cm (always 1 cm).

The results obtained are summarised in the following tables:

**Table 6 - 3,5-dicaffeoyl-4-malonylquinic acid** (m.w. 602)

| | | ***Spec. abs. (1 µg/ml) mAU*** | ***Epsilon*** |
|---|---|---|---|
| pH 5.0 | λ Max 325 nm 330 nm | 51.3 50.3 | 30.602 29.972 |
| pH 1.0 | λ Max 326 nm 330 nm | 47.9 47.2 | 28.539 28.138 |

**Table 7** - **PARSOL MCX (ethyl-hexyl methoxycinnamate)** (m.w. 290) (oil) sol. EtOH/$H_3PO_4$ (1/1)

| | | ***Spec. abs. (1 µg/ml) mAU*** | ***Epsilon*** |
|---|---|---|---|
| pH 5.0 | λ Max 312 nm | 48.85 | 14.166 |
| in abs. EtOH | λ Max 310 nm | 87.26 (*) | 25.305 |
| (*) E 1% at 310 nm in abs. EtOH = (87.26 x $10^4$) UA 872 (literature value 865) | | | |

At λ Max, the data obtained show a higher epsilon value for the compound 3,5-dicaffeoyl-4-malonylquinic acid compared to the commercially available compound Parsol MCX. These results indicate that said compound may act as an optimal natural sun screen.

**EXAMPLE 7**

**THE DEPIGMENTING ACTIVITY OF 3,5-DICAFFEOYL-4-MALONYLQUINIC ACID**

The bleaching activity of the compound obtained by cell culture of *Centella asiatica* has been determined by evaluating the inhibition of melanin production, stimulated by the addition of MSH (melanocyte stimulating hormone) in mouse B16F1 melanoma cells. The test has been prepared by seeding mouse B16F1 melanoma cells in DMEM culture medium + 10% FBS in 25 $cm^2$ flasks. On reaching 80% confluency, the culture medium has been replaced with conditioned medium (containing 100 nM MSH and the test compounds). The cells in the conditioned medium have been incubated for 24 hours at 37°C and 5% $CO_2$. On termination of incubation, the cells have been washed twice with PBS and then detached using a Trypsin-EDTA solution. After washing twice with PBS, the detached cells have been resuspended in the same solution. A small aliquot has been removed from the suspension for the purpose of conducting the MTT cell viability test, while the remainder has been used to extract the cell melanin content. The melanin content has been determined by spectrophotometric measurements at 405 nm, and calculated by means of a calibration curve obtained using a melanin standard. The optical density value obtained has been normalised for the number of viable cells obtained by means of the MTT test. The percentage inhibition of melanin production has been calculated from the percentage ratio between the value obtained from the cells treated with the extracts, and the value obtained from the control cells, treated with MSH alone.

The results obtained from testing the compound at a concentration of 1 mg/ml compared to kojic acid at the same concentration, are reported in table 8 below:

TABLE 8

| Compounds (1 mg/mL) | % inhibition | % inhibition vs AK |
|---|---|---|
| Kojic acid | 70 | 100 |
| 3,5-dicaffeoyl-4-malonylquinic acid | 33.8 | 48 |

The compound 3,5-dicaffeoyl-4-malonylquinic acid, obtained from cell culture of *Centella asiatica,* exerts strong inhibitory activity with regard to melanin production in B16F1 melanoma cells.

In particular, compared to kojic acid, considered as a reference, inhibition reaches values close to 50%.

**EXAMPLE 8**

**THE ANTIVIRAL ACTIVITY OF 3,5-DICAFFEOYL-4-MALONYLQUINIC ACID**

The antiviral activity of 3,5-dicaffeoyl-4-malonylquinic acid towards type-2 HSV virus has been assayed using a Vero cell model. The assay has been conducted using acyclovir as a comparator.

The method used has been the following:

Vero cells have been seeded into 96 well plates at a concentration of $5x10^4$ cells/well. After 4 hours incubation at 37°C in 5% $CO_2$, the cells have been infected with strain 333 of the type-2 Herpes simplex virus (HSV) at a multiplicity of infection (MOI) of 0.1 infective viral particles/cell. Various concentrations of 3,5-dicaffeoyl-4-malonylquinic acid (CA1) have been added in triplicate to the cell monolayers. Some wells have been infected in the absence of test compound (POSITIVE CONTROL), others have not been infected and have not been treated with the test compound (NEGATIVE CONTROL), others still have been treated with various concentrations of acyclovir (ACYC), representing the comparator substance. The infected cells have then been incubated in medium plus 2% FBS for 72 hours. On completion of said incubation period, the medium has been removed from each well by aspiration. Following washing in PBS and aspiration, 50 μl of a solution consisting of 100 μl PMS and 1 mg/ml XTT has been added to each well. The plates have been incubated for a further 2-4 hours in the dark, in order to allow formation of the formazan. The optical density has been measured using an ELISA plate reader set at a wavelength of 450 nm and 630 nm.

The results obtained indicate that, at a concentration of 200 μg/ml, 3,5-dicaffeoyl-4-malonylquinic acid (CA1) has good antiviral activity if compared to the 0 value (positive control of cells infected in the absence of test substance) and is not cytotoxic up to a concentration of 1000 μg/ml.

The diagram in figure 2 shows the data obtained from the antiviral activity assay.

**EXAMPLE 9**

**THE METALLOPROTEINASE INHIBITORY ACTIVITY OF 3,5-DICAFFEOYL-4-MALONYLQUINIC ACID**

Metalloproteinase inhibitory activity has been evaluated by preparation of an *in vitro* assay known as the "3D model" envisaging the use of a type-1 collagen gel and BALB 3T3 fibroblast cells. In this assay model, fibroblasts acquire the property of secreting metalloproteinases (MMP), such as collagenase, gelatinase, elastase etc. through modulation by cytokines or other inflammatory mediators (Lim H., Kim HP, Planta Medica, 2007, 12:1267-1274). Balb 3T3 cells ($4*10^6$ cells/ms) are inoculated in a culture medium (DMEM + 10% FBS) containing a type 1 collagen 3D gel supplemented with increasing concentrations of the test compound. After incubation for 48 hours at 37°C in 5% $CO_2$, the collagen content is measured by means of an indirect method (hydroxyproline content); in parallel, the effect of treatment on cell viability, or on the growth inhibitory effect, is also assessed. The result is expressed as the %age of hydroxyproline present in the 3D gel, compared to the control, or untreated, culture (NB: the collagen content in the absence of cells is reported as positive control). The data obtained is reported in figure 3. From the results obtained, it is evident that treatment with 3,5 dicaffeoyl-4-malonylquinic acid is effective up to a concentration of 400 μg/ml. For higher concentrations, the collagen content increases purely as a result of the reduced number of cells.

With regard to its high antioxidant activity, as described in example 3, the compound 3,5-dicaffeoyl-4-malonylquinic acid is a valid tool in the prevention and treatment of disease states associated with damage due to free radicals such as: anoxic trauma and nervous system inflammatory based disorders, degenerative nervous system disorders also associated with ageing or autoimmune and inflammatory component based, damage due to reperfusion and cardiovascular disorders, atherosclerosis and muscle damage, dismetabolic disorders such as type-I and type-II diabetes mellitus and the neurological, vascular and ophthalmic complications thereof, toxic disorders such as alcoholism and the neurological complications thereof in addition to cirrhosis of the liver, peripheral neuropathies including toxic forms, skin

disorders, including cellulitis and mucosal disorders, including the oropharyngeal, gastrointestinal and vaginal mucosa, dental disorders, respiratory disorders, articular disorders, cancer; ophthalmic disorders, hearing loss and diminished immune response.

Thanks to the marked anti-inflammatory activities described in example 4, the compound is a valid tool in the prevention and treatment of disorders associated with chronic and acute inflammatory states.

The antimicrobial activities of the compound, as demonstrated in example 5, make it a valid tool in the protection against and treatment of disease states associated with lesions of the epidermis and skin resulting from burns, cuts or lesions and mechanical trauma, diabetic related sores and bedsores, aiding the wound-healing and tissue-repair processes.

As shown in example 7, *3,5-dicaffeoyl-4-malonylquinic acid* shows high melanin production inhibitory activity. This activity may be exploited in order to bring about bleaching effects on the skin.

3,5-dicaffeoyl-4-malonylquinic acid shows a marked capacity to absorb UV radiation, as shown in example 6. Thanks to this characteristic, the compound may be used effectively as a sun screen.

Due to the marked antiviral activities shown in example 8, 3,5-dicaffeoyl-4-malonylquinic acid may be justifiably used in all pathologies associated with viruses such as Herpes.

Furthermore, as reported in example 9, 3,5-dicaffeoyl-4-malonylquinic acid shows high metalloproteinase inhibitory activity. This characteristic may be widely exploited in the prevention of skin ageing and scar formation processes.

The doses, times and routes of administration of the treatment will be selected on the basis of the type, stage, severity and location of manifestation of the pathology. For all the pathologies mentioned, systemic and oral administration are indicated, but additionally also topical and transdermal, and in any case so as to make the active ingredient maximally available. For the oral formulations, administration in the form of tablets, lozenges and capsules, but also powders and suspensions are preferred: for topical treatment, gels, creams, ointments and solutions compatible with dermal and mucosal use are preferred, in addition to eye washes for administration into the conjunctival sac and nebulised forms for use by inhalation. The injectable forms are formulated using solvents for pharmaceutical use, compatible with intravenous, intramuscular and subcutaneous administration.

The therapeutic dose varies, depending on the patients age, weight, sex and type of pathology, between 0.1 mg and 2 g per day and preferably between 5 and 150 mg per day, taken in a single dose or in 2-4 doses or in slow release form, depending on the patients therapeutic need, and for periods of time varying between 1 and 120 days.

At appropriate concentrations, the same compound may be formulated in the form of a supplement, to be taken orally for prevention, or as aids in the treatment of changes resulting from disreactive states in humans or in veterinary medicine. The compound, active at the appropriate concentrations, and in appropriate formulations, may be likewise used in cosmetics and trichological treatments.

## Claims

1. *Centella asiatica* cell culture extracts comprising 3,5-dicaffeoyl-4-malonylquinic acid of formula (I) in quantities exceeding 10% by weight.

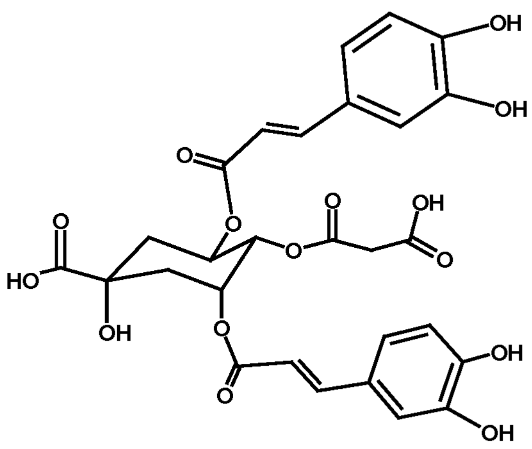

(I)

2. Method for the preparation of 3,5-dicaffeoyl-4-malonylquinic acid of formula (I), wherein:

   1) a *Centella asiatica* cell culture is prepared by means of a method including:

i) the attainment of calluses from *Centella asiatica* plant tissue,
ii) the stabilisation of the cell line obtained from callus tissue,
iii) the selection of a culture with high 3,5-dicaffeoyl-4-malonylquinic acid content,
iv) the transfer of the stabilised and selected cell line in liquid culture medium, and the production of biomass in suitable fermenters; and

2) an extraction method is performed on said *Centella asiatica* cell culture including:

i) forming a homogenised suspension of said biomass at pH 6 to 12, preferably at pH 8,
ii) eliminating the corpuscular part,
iii) extracting the resulting solution, at a pH of between approx. 3 and 4 with a solvent capable of dissolving 3,5-dicaffeoyl-4-malonylquinic acid, or
iv) extracting the resulting solution, at a pH of between approx. 3 and 4, with hydrophobic interaction resin.

**Patentansprüche**

1. *Centella asiatica* Zellkulturextrakte, umfassend 3,5-Dicaffeoyl-4-malonylchinasäure der Formel (I) in Mengen, die 10 Gew.-% übersteigen.

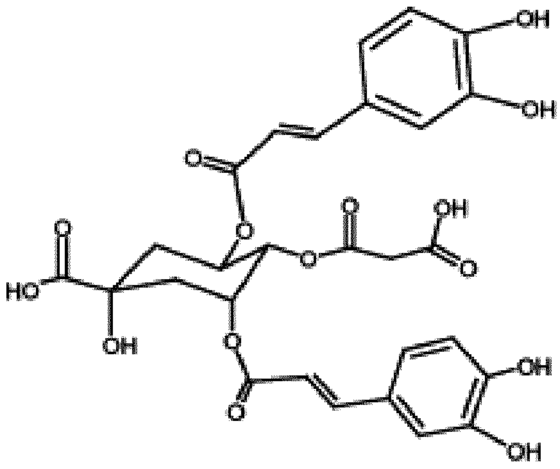
(I)

2. Verfahren zur Herstellung von 3,5-Dicaffeoyl-4-malonylchinasäure der Formel (I), wobei:

1) eine *Centella asiatica* Zellkultur mittels eines Verfahrens hergestellt wird, das Folgendes enthält:

i) das Erzielen von Kallussen aus dem Pflanzengewebe von *Centella asiatica,*
ii) die Stabilisierung der aus dem Kallusgewebe erhaltenen Zelllinie,
iii) die Auswahl einer Kultur mit hohem Gehalt an 3,5-Dicaffeoyl-4-malonylchinasäure
iv) der Transfer der stabilisierten und ausgewählten Zelllinie in flüssiges Kulturmedium und die Herstellung einer Biomasse in geeigneten Fermentern; und

2) ein Extraktionsverfahren an der *Centella asiatica* Zellkultur durchgeführt wird, einschließlich:

i) des Bildens einer homogenisierten Suspension der Biomasse bei einem pH-Wert von 6 bis 12, bevorzugt einem pH-Wert von 8,
ii) des Eliminierens des korpuskularen Teils,
iii) des Extrahierens der resultierenden Lösung bei einem pH-Wert von zwischen ungefähr 3 und 4 mit einem Lösungsmittel, das in der Lage ist, die 3,5-Dicaffeoyl-4-malonylchinasäure aufzulösen oder
iv) des Extrahierens der resultierenden Lösung bei einem pH-Wert von zwischen ungefähr 3 und 4 mit hydrophobem Interaktionsharz.

## Revendications

1. Extraits de culture cellulaire de *Centella asiatica* comprenant de l'acide 3,5-dicaffeoyl-4-malonylquinique de formule (I) en quantités excédant 10% en poids.

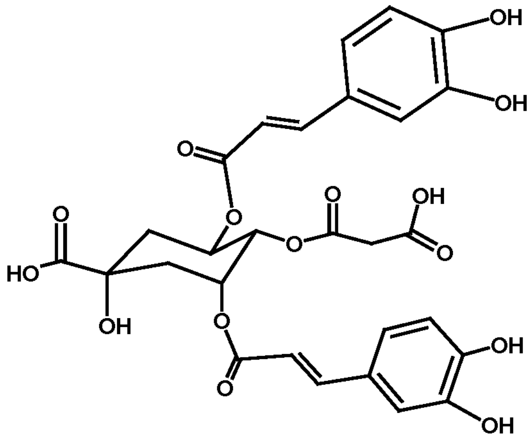
(I)

2. Procédé de préparation d'acide 3,5-dicaffeoyl-4-malonylquinique de formule (I), dans lequel:

   1) une culture cellulaire de *Centella asiatica* est préparée au moyen d'une méthode comprenant :

      i) l'obtention de cals à partir de tissu végétal de *Centella asiatica,*
      ii) la stabilisation de la lignée cellulaire obtenue à partir de tissu de cals,
      iii) la sélection d'une culture avec une quantité d'acide 3,5-dicaffeoyl-4-malonylquinique élevée,
      iv) le transfert de la lignée cellulaire stabilisée et sélectionnée dans un milieu de culture liquide, et la production de biomasse dans des fermenteurs adaptés ; et

   2) un processus d'extraction est réalisé sur ladite culture cellulaire de *Centella asiatica* cell comprenant :

      i) la formation d'une suspension homogénéisée de ladite biomasse à un pH de 6 à 12, de préférence à pH 8,
      ii) l'élimination des parties corpusculaires,
      iii) l'extraction de la solution obtenue, à un pH approximativement compris entre 3 et 4 avec un solvant capable de dissoudre l'acide 3,5-dicaffeoyl-4-malonylquinique, ou
      iv) l'extraction de la solution obtenue, à un pH approximativement compris entre 3 et 4, avec une résine d'interaction hydrophobe.

Figure 1: chromatography profile of *Centella asiatica* extract

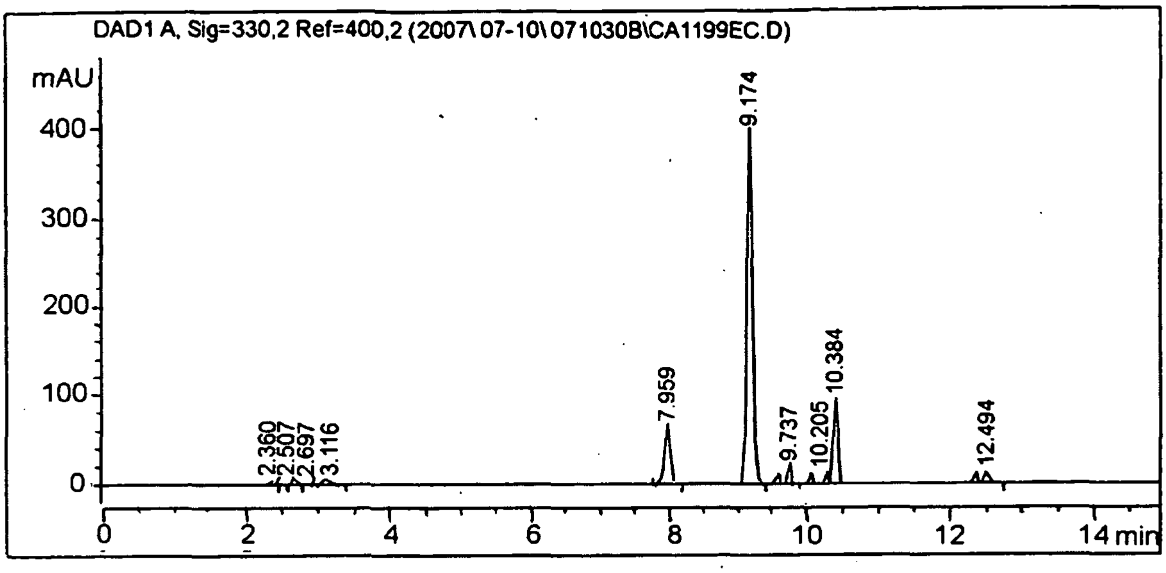


Figure 2: anti HSV2 antiviral activity of 3,5 dicaffeoyl-4-malonylquinic acid.

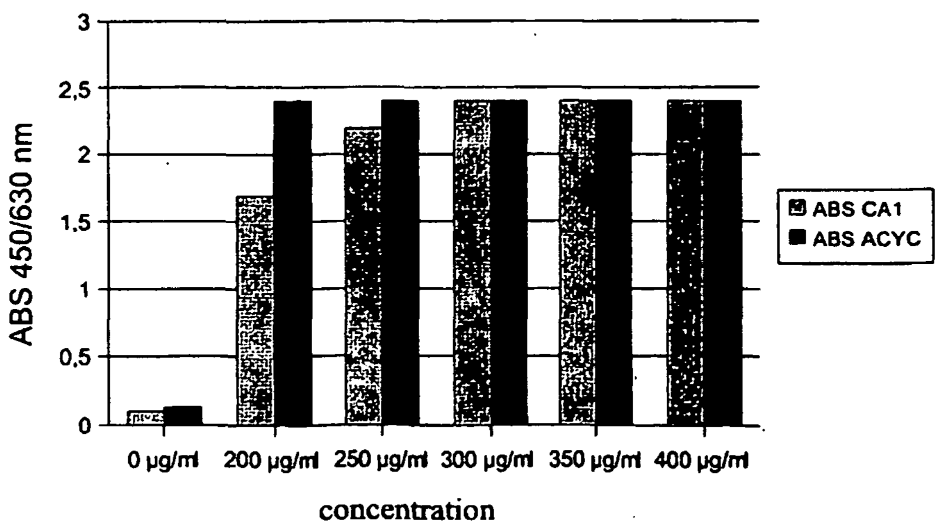

Figure 3: metalloproteinase inhibitory activity of 3,5 dicaffeoyl-4-malonylquinic acid.

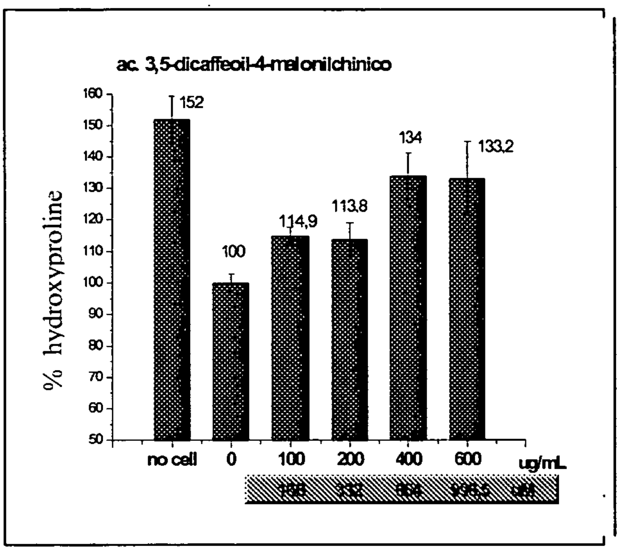

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1027878 A


### Non-patent literature cited in the description


- **KARTNING T.** Herbs, Spices and medicinal plants. Oryx Press Arizona, 1998, vol. 3
- **BELCARO G. et al.** *Angiology,* 1990, vol. 41, 12
- **BELCARO G. et al.** *Curr. Ther. Res.,* 1990, vol. 47, 421
- **TOYOKUNI S. et al.** *FEBS Letters,* 1995, vol. 358, 1-3
- **OCHIAI T. et al.** *Neuroscience Letters,* 2004, vol. 362, 61-64
- **MERTENS et al.** *Circulation,* 2003, vol. 107, 1640-1646
- **SALVEMINI D. ; CUZZOCREA S.** *Free Radical Biology & Medicine,* 2002, vol. 33-9, 1173-1185
- **YANN C. et al.** *Pharmacological reports,* 2005, vol. 57, 97-107
- **PAWLICZAK R.** *Pol. Merkuriusz Lek.,* 2003, vol. 14, 493-6
- **LEIRO J. et al.** *J. Leukoc. Biol.,* 2004, 75
- **ZHANG YUFENG.** Characterization of phenolic compounds in Erigeron breviscapus by liquid chromatography coupled to electrospray ionization mass spectrometry. *Rapid Commun Mass Spectrom,* 2007, vol. 21 (18), 2971-84
- **HONG LIU.** *Effects of Erigeron breviscapus ethanol extract on neuronal oxidative injury induced by superoxide radical Fitoterapia,* December 2005, vol. 76 (7-8), 666-70
- Xanthine oxidase inhibitory activity of Vietnamese medicinal plants. **MAI THANH THI NGUYEN.** Biol Pharm Bull. scientific publication, September 2004, vol. 27, 1414-21
- **SATAKE TOSHIKO.** The anti-thrombotic active constituents from Centella asiatica. *Biol Pharm Bull.,* May 2007, vol. 30 (5), 935-40
- **J. M. SOLET.** Centella asiatica (L.) Urban, (Pennywort): Cell Culture, Production of Terpenoids, and Biotransformation Capacity. *Biotechnology in Agriculture and Forestry,* vol. 41, 81-96
- Effects of Precursor Supplementation on the Production of Triterpenes by Centella asiatica Callus Cultures. **ANNA LING PICK KIANG.** Pakistan Jownal of Biological Sciences. The scientific publication, 2005, vol. 8, 1160-1169
- Development of Growth Medium for Centella Asiatica Cell Culture via Response Surface Methodology. American Journal of Applied Sciences. The scientific publication, 2004, vol. 1, 215-219
- Optimization and elucidation of interactions between ammonium, nitrate and phosphate in Centella asiatica cell culture using response surface methodology. Biotechnology and Bioprocess Engineering. The scientific publication, June 2005, vol. 10, 192-197
- Biotechnology and Bioprocess Engineering. The scientific publication, June 2006, vol. 11, 223-229
- **SCHMAUDER H.P. ; DOEBEL P.** *Acta Biotechnolo.,* 1990, vol. 10, 501-516
- **J. BUENGER et al.** *International Journal of Cosmetic Science,* 2006, vol. 28, 135-146
- **LIM H. ; KIM HP.** *Planta Medica,* 2007, vol. 12, 1267-1274